# EUROPÄISCHE PATENTSCHRIFT

(11) **EP 2 897 540 B1**
(45) Veröffentlichungstag und Bekanntmachung des Hinweises auf die Patenterteilung: **13.12.2017**
(21) Anmeldenummer: 13766255.7
(22) Anmeldetag: 17.09.2013
(51) Int. Cl.: A61B 17/34

(54) **CHIRURGISCHE SCHUTZVORRICHTUNG FÜR EIN CHIRURGISCHES DICHTELEMENT UND CHIRURGISCHES ABDICHTUNGSSYSTEM**
SURGICAL PROTECTIVE DEVICE FOR A SURGICAL SEALING ELEMENT AND SURGICAL SEALING SYSTEM
DISPOSITIF DE PROTECTION CHIRURGICAL POUR UN ÉLÉMENT D'ÉTANCHÉITÉ CHIRURGICAL ET SYSTÈME D'ÉTANCHÉITÉ CHIRURGICAL

(30) Priorität: 19.09.2012 DE 102012108809
(43) Veröffentlichungstag der Anmeldung: 29.07.2015
(73) Patentinhaber: Aesculap AG, 78532 Tuttlingen (DE)
(72) Erfinder: SAUTER, Wolfgang, 78603 Renquishausen (DE)
(74) Vertreter: Hoeger, Stellrecht & Partner Patentanwälte mbB
(86) Internationale Anmeldenummer: PCT/EP2013/069247
(87) Internationale Veröffentlichungsnummer: WO 2014/044665

(56) Entgegenhaltungen:
- EP-B1- 2 138 112
- DE-U1-202008 009 527
- US-A1- 2005 070 851
- US-A1- 2010 274 193
- US-A1- 2011 251 560

## Beschreibung

Die vorliegende Erfindung betrifft eine chirurgische Schutzvorrichtung für ein chirurgisches, eine erweiterbare Einführöffnung aufweisendes Dichtelement eines chirurgischen, eine Trokarhülse umfassenden Abdichtungssystems, welche Schutzvorrichtung einen an der Trokarhülse, an einem Teil derselben oder am Dichtelement anordenbaren, ringförmigen oder im Wesentlichen ringförmigen und eine Durchbrechung definierenden Grundkörper mit mehreren in Umfangsrichtung angeordneten und parallel oder auf eine Längsachse der Schutzvorrichtung hin weisenden Schutzelementen umfasst, wobei die Schutzvorrichtung einstückig ausgebildet ist.

Ferner betrifft die Erfindung ein chirurgisches Abdichtungssystem umfassend eine Trokarhülse, ein an der Trokarhülse oder einem Teil derselben gehaltenes chirurgisches, eine erweiterbare Einführöffnung aufweisendes Dichtelement zum Abdichten der Einführöffnung beim Einführen eines chirurgischen Instruments und eine chirurgische Schutzvorrichtung für das Dichtelement.

Eine chirurgische Schutzvorrichtung sowie ein chirurgische Abdichtungssystem sind beispielsweise aus der DE 20 2008 009 527 U1 bekannt, welche als nächstliegender Stand der Technik angesehen werden kann. Die Schutzvorrichtung dient dazu, eine Beschädigung des Dichtelements des Abdichtungssystems beim Einführen chirurgischer Instrumente, oder auch eines Obturators zum Verschließen der Trokarhülse, zu verhindern. Ein Problem stellen sowohl die Herstellung der Schutzvorrichtung als auch das Positionieren derselben an beziehungsweise in der Dichtung dar. So müssen bei allen Schutzvorrichtungen, die mehrere Schutzelemente umfassen, diese einander überlappend angeordnet werden, damit die Schutzvorrichtung an eine Innenseite des Dichtelements herangeführt werden kann.

Aus der US 2011/0251560 A1 ist eine plissierte Schutzvorrichtung für einen Trokar bekannt. In der US 2010/274193 A1 ist eine Kanülendichtung beschrieben. Ein Trokargehäuse ist in der US 2005/0070851 A1 offenbart. Ferner ist aus der EP 2 138 112 B1 eine Irisversiegelung für die Single-Incision-Chirurgie bekannt.

Es ist daher eine Aufgabe der vorliegenden Erfindung, eine Überlappung der Schutzelemente der Schutzvorrichtung zu vereinfachen.

Diese Aufgabe wird bei einer chirurgischen Schutzvorrichtung der eingangs beschriebenen Art erfindungsgemäß dadurch gelöst, dass jedes der Schutzelemente bezogen auf eine Ebene, welche eine vom Grundkörper definierte Längsachse enthält, asymmetrisch ausgebildet ist, dass die Schutzelemente erste Enden aufweisen, die am Grundkörper angeordnet sind, und dass jedes Schutzelement zwei sich vom ersten Ende weg erstreckende Ränder aufweist, dass der eine Rand vom Schutzelement weg weisend konvex gekrümmt ist und dass der andere Rand vom Schutzelement weg weisend konkav gekrümmt ist.

Durch die asymmetrische Ausgestaltung der Schutzelemente kann zum einen insbesondere eine sichere Abdeckung des Dichtelements gewährleistet und zum anderen insbesondere auch vermieden werden, ein unnötig hohes beziehungsweise dickes Materialpaket durch Mehrfachüberlappungen zu erzeugen. Auch das Übereinanderlegen der Schutzelemente zur Überlappung derselben wird insbesondere durch die asymmetrische Form der Schutzelemente vereinfacht. Günstigerweise weisen die Schutzelemente erste Enden auf, die am Grundkörper angeordnet sind. So können insbesondere zweite beziehungsweise freie Enden der Schutzelemente relativ zum Grundkörper frei bewegt werden. Gemäß der Erfindung ist vorgesehen, dass jedes Schutzelement zwei sich vom ersten Ende weg erstreckende Ränder aufweist, dass der eine Rand vom Schutzelement weg weisend konvex gekrümmt ist und dass der andere Rand vom Schutzelement weg weisend konkav gekrümmt ist. Auf diese Weise lassen sich definiert asymmetrische Schutzelemente ausbilden, die eine im Vergleich zum Stand der Technik optimierte Überlappung ermöglichen, und zwar unabhängig davon, wie weit das Dichtelement in Abhängigkeit eines Durchmessers eines eingeführten Instruments geöffnet ist. Die Herstellung der Schutzvorrichtung wird deutlich vereinfacht dadurch, dass diese einstückig ausgebildet ist. So kann vermieden werden, dass die Schutzelemente einzeln mit dem Grundkörper verbunden werden müssen.

Besonders einfach und sicher handhabbar wird die Schutzvorrichtung, wenn der Grundkörper im Wesentlichen in Form einer ringförmigen Hülse ausgebildet ist. So kann diese beispielsweise einen maximalen Innendurchmesser definieren, welcher von dem Dichtelement abdichtbar ist. Mit anderen Worten können keine Instrumente durch das Abdichtungssystem eingeführt werden, die einen größeren Außendurchmesser aufweisen als der Innendurchmesser des Grundkörpers.

Eine besonders einfache und sichere Überlappung der Schutzelemente kann insbesondere dadurch erreicht werden, dass die Schutzelemente von der Längsachse weg weisend schwach konvex gekrümmt sind. Insbesondere kann eine Krümmung der Schutzelemente einer Krümmung einer den Grundkörper definierenden Wand der Hülse entsprechen.

Vorzugsweise sind die Schutzelemente an einer in distaler Richtung weisenden Randfläche des Grundkörpers angeordnet. Auf diese Weise lässt sich ein besonders kompakter Aufbau der Schutzvorrichtung erzeugen.

Um eine maximale Beweglichkeit der Schutzelemente relativ zum Grundkörper zu erreichen, ist es vorteilhaft, wenn die ersten Enden der Schutzelemente einander nicht überlappen.

Günstig ist es, wenn die ersten Enden der Schutzelemente voneinander beabstandet sind. So kann eine Beweglichkeit der Schutzelemente relativ zum Grundkörper erhöht werden.

Vorzugsweise entspricht ein Abstand der ersten Enden benachbarter Schutzelemente mindestens einer Dicke der Schutzelemente und/oder des Grundkörpers. Mit einem solchen Abstand der ersten Enden benachbarter Schutzelemente voneinander kann sichergestellt werden, dass eine möglichst große Beweglichkeit, insbesondere eine Verschwenkbarkeit, der Schutzelemente in Richtung auf die Längsachse hin möglich ist.

Günstigerweise definieren die ersten Enden der Schutzelemente einen Kreisbogenausschnitt. Sie können somit insbesondere direkt als eine Verlängerung einer Wand der Hülse ausgebildet werden.

Vorteilhaft ist es, wenn die ersten Enden der Schutzelemente konzentrisch zur Längsachse angeordnet sind. Dies erlaubt es insbesondere, den hülsenförmigen Grundkörper der Schutzvorrichtung durch die Schutzelemente quasi in distaler Richtung zu verlängern.

Die Herstellung der Schutzvorrichtung wird vereinfacht, wenn die Schutzelemente in einer Grundstellung nach der Herstellung in radialer Richtung über die in distaler Richtung weisende Randfläche von der Längsachse weg weisend abstehen. Insbesondere kann die Schutzvorrichtung derart ausgebildet sein, dass die Schutzelemente einander in der Grundstellung nicht überlappen. So kann die Schutzvorrichtung beispielsweise in gewünschter Weise und definiert aus einem Kunststoff durch Spritzgießen hergestellt werden.

Um ein optimales Anlegen der Schutzelemente an das zu schützende Dichtelement zu ermöglichen, ist es vorteilhaft, wenn die Schutzelemente in Form von Lamellen geformt sind, welche eine Dicke aufweisen, welche maximal einer Dicke der den Grundkörper definierenden Hülse entspricht.

Sowohl die Konstruktion als auch die Herstellung der Schutzvorrichtung lassen sich insbesondere dadurch weiter vereinfachen, dass alle Schutzelemente identisch ausgebildet sind.

Grundsätzlich wäre es zwar denkbar, einander benachbarte Schutzelemente spiegelsymmetrisch zu einer die Längsachse enthaltenden Ebene anzuordnen beziehungsweise auszubilden. Vorteilhaft ist es jedoch, wenn die konvex gekrümmten Ränder der Schutzelemente in der Grundstellung auf die konkav gekrümmten Ränder direkt benachbarter Schutzelemente hin weisen. Auf diese Weise kann insbesondere ein Abstand zwischen den konvex und konkav gekrümmten Rändern benachbarter Schutzelemente minimiert werden, und zwar bereits in der Grundstellung. Auch kann so insbesondere eine Überlappung benachbarter Schutzelemente optimiert werden, beispielsweise auch dahingehend, dass eine mehrfache Überlappung von Schutzelementen, also mehr als zwei überlappende Schutzelemente, vermieden wird. Dies bedeutet insbesondere, dass ein Radialstrahl von der Längsachse weg nie mehr als beispielsweise zwei Schutzelemente durchstößt.

Wie bereits erwähnt, ist es für die Herstellung der Schutzvorrichtung vorteilhaft, wenn sich die Schutzelemente in der Grundstellung nicht überlappen.

Günstig ist es, wenn die Schutzelemente ein zweites, vom Grundkörper weg weisendes, insbesondere freies, Ende aufweisen. Diese Ausgestaltung ermöglicht insbesondere eine für den Einsatz der Schutzvorrichtung erforderliche Beweglichkeit.

Vorteilhafterweise erstreckt sich das zweite Ende zwischen den sich von den vom ersten Ende weg erstreckenden Rändern. Damit liegt das zweite Ende der Schutzelemente insbesondere dem ersten Ende im Wesentlichen gegenüber. Die konvex und konkav gekrümmten Ränder der Schutzelemente bilden somit seitliche Begrenzungen.

Günstig ist es, wenn das zweite Ende eine Endrandfläche definiert, welche im Wesentlichen eine vom Grundkörper weg weisend schwach konvex gekrümmte Grundform aufweist, und wenn die Endrandfläche einen vom Grundkörper weg weisend konkav gekrümmten Randabschnitt umfasst. Diese Ausgestaltung hat insbesondere den Vorteil, dass der konkav gekrümmte Randabschnitt auf die Längsachse hin weisen kann, wenn die Schutzelemente in Richtung auf die Längsachse hin verschwenkt sind, so dass die konkav gekrümmten Randabschnitte aller Schutzelemente zusammen eine im Wesentlichen kreisförmige Öffnung der Schutzvorrichtung definieren können, welche dann die Längsachse umgibt.

Vorzugsweise nimmt eine Breite der Schutzelemente parallel zu einer die ersten Enden enthaltenden Ebene ausgehend vom ersten Ende in Richtung auf das zweite Ende hin zu. So kann insbesondere unabhängig von einer Stellung der Schutzelemente, also insbesondere unabhängig von einer Verschwenkung derselben relativ zum Grundkörper auf die Längsachse hin, eine definierte Überlappung erreicht werden, um das Dichtelement des Abdichtungssystems stets sicher zu schützen. Insbesondere hat diese Ausgestaltung den Vorteil, dass auch noch eine sichere Überlappung und damit ein Schutz des Dichtelements gewährleistet ist, wenn Instrumente mit sehr großem Durchmesser durch das Abdichtungssystem eingeführt.

Grundsätzlich wäre es denkbar, dass die Breite der Schutzelemente bis zum zweiten Ende hin zunimmt. Vorteilhaft ist es jedoch, wenn die Breite der Schutzelemente zwischen dem ersten Ende und der Endrandfläche ein Maximum aufweist. Eine solche Ausgestaltung ermöglicht insbesondere eine Form der Schutzelemente zu realisieren, die unabhängig von einer Schwenkstellung derselben relativ zum Grundkörper stets nur eine Überlappung benachbarter Schutzelemente sicherstellt.

Die Überlappung kann insbesondere dadurch weiter optimiert werden, dass das Maximum der Breite der Schutzelemente dem zweiten Ende näher als dem ersten Ende der Schutzelemente ist. So kann insbesondere auch noch bei sehr großen Instrumentendurchmessern eine Überlappung und damit ein Schutz des Dichtelements gewährleistet werden.

Um eine optimale Beweglichkeit zwischen den Schutzelementen und dem Grundkörper zu erreichen, ist es günstig, wenn die Schutzelemente in einem Übergangsbereich zum Grundkörper gelenkig und/oder scharnierartig mit dem Grundkörper verbunden oder gekoppelt sind.

Besonders einfach herzustellen ist die Schutzvorrichtung, wenn die Schutzelemente und der Grundkörper über ein Filmscharnier miteinander verbunden sind. So kann die Schutzvorrichtung insbesondere auch einstückig ausgebildet werden.

Vorzugsweise sind die Schutzelemente von der Grundstellung in eine Schutzstellung in Richtung auf die Längsachse hin verschwenkbar. So kann insbesondere eine definierte Beweglichkeit zwischen den Schutzelementen und dem Grundkörper ermöglicht werden.

Günstigerweise überlappen sich benachbarte Schutzelemente in einer Schutzstellung teilweise. Dadurch kann unabhängig von einer Schwenkstellung der Schutzelemente in Richtung auf die Längsachse hin ein Schutz des Dichtelements gewährleistet werden. Insbesondere können die Schutzelemente in der Schutzstellung in Richtung auf die Längsachse hin weisen. In der Schutzstellung können die Schutzelemente jedoch insbesondere unterschiedliche Schwenkwinkel zwischen sich und dem Grundkörper definieren, wobei der Verschwenkwinkel abhängt von einem Durchmesser des in das Abdichtungssystem eingeführten Instruments sowie einer Einführrichtung desselben bezogen auf die Längsachse des Abdichtungssystems.

Gemäß einer weiteren bevorzugten Ausführungsform der Erfindung kann vorgesehen sein, dass die Schutzelemente in der Schutzstellung höchstens einen gemeinsamen maximalen Innendurchmesser definieren, der einem Innendurchmesser des Grundkörpers entspricht. Auf diese Weise kann sichergestellt werden, dass auch beim Einführen von Instrumenten mit einem Außendurchmesser, der im Wesentlichen dem Innendurchmessers des Grundkörpers entspricht, das Dichtelement noch vollständig beziehungsweise im Wesentlichen vollständig durch die Schutzelemente der Schutzvorrichtung geschützt ist.

Ferner ist es vorteilhaft, wenn die Schutzelemente, wenn sie sich maximal überlappen, in der Schutzstellung einen minimalen Innendurchmesser definieren und wenn dabei die konkav gekrümmten Randabschnitte der Endrandflächen konzentrisch oder im Wesentlichen konzentrisch zur Längsachse ausgerichtet und dieser maximal angenähert sind. Die Schutzelemente definieren somit in einer maximal überlappenden Schutzstellung eine von den konkav gekrümmten Randabschnitten der Endrandflächen begrenzte Öffnung, die dann vorzugsweise einen Innendurchmesser definiert, welcher einem kleinsten Innendurchmesser des Dichtelements entspricht. Damit ist das Dichtelement im ungedehnten Zustand, also ohne dass ein Instrument in das Abdichtungssystem eingeführt ist, maximal gut geschützt.

Um unnötig hohe Materialpakete beim Einsatz der Schutzvorrichtung zu vermeiden, ist es günstig, wenn sich die Schutzelemente in der Schutzstellung, unabhängig davon, welchen Innendurchmesser sie dabei mit ihren zweiten Enden definieren, nur derart überlappen, dass ausschließlich einander benachbarte Schutzelemente übereinander liegen. Auf diese Weise kann insbesondere eine Dreifachüberlappung von Schutzelementen sowie eine Überlappung von noch mehr Schutzelementen vermieden werden.

Eine von den zweiten Enden der Schutzelemente definierte Öffnung lässt sich insbesondere dadurch mit besonders geringem Kraftaufwand öffnen, wenn der konkav gekrümmte Rand der Schutzelemente den konvex gekrümmten Rand auf einer von der Längsachse weg weisenden Außenseite des direkt benachbarten Schutzelements überdeckt oder umgekehrt. Mit anderen Worten kann beispielsweise der konkav gekrümmte Rand eines Schutzelements innen oder außen am benachbarten Schutzelement anliegen. Diese Bedingung ist für alle Schutzelemente der Schutzvorrichtung gleichermaßen zu erfüllen. Weisen die Schutzelemente ferner optional eine reibungsmindernde äußere Oberfläche auf, beispielsweise durch Aufbringen einer entsprechenden Beschichtung, kann so ein Öffnen und Schließen der Schutzvorrichtung weiter erleichtert werden.

Zum Anbringen der Schutzvorrichtung am Abdichtungssystem ist vorteilhaft, wenn sie eine am Grundkörper angeordnete Verbindungseinrichtung zum Verbinden der Schutzvorrichtung mit dem chirurgischen Dichtelement oder dem chirurgischen Abdichtungssystem umfasst. Mit anderen Worten kann die Schutzvorrichtung insbesondere direkt mit dem Dichtelement und über dieses indirekt mit einer Dichtelementhalterung des Abdichtungssystems verbunden werden, beispielsweise dauerhaft unlösbar oder nur temporär.

Eine besonders einfache Kopplung der Schutzvorrichtung mit dem Dichtelement beziehungsweise dem Abdichtungssystem kann insbesondere dadurch erreicht werden, dass die Verbindungseinrichtung mindestens ein in radialer Richtung vom Grundkörper abstehendes Verbindungselement umfasst.

Um eine möglichst stabile und definierte Verbindung zwischen der Schutzvorrichtung und dem Dichtelement beziehungsweise dem Abdichtungssystem herstellen zu können, ist es günstig, wenn mehrere, in Umfangsrichtung voneinander beabstandete Verbindungselemente vorgesehen sind.

Vorteilhafterweise sind die Verbindungselemente gleichmäßig über den Umfang des Grundkörpers verteilt angeordnet. So können auf die Schutzvorrichtung einwirkende Kräfte gleichmäßig auf das Dichtelement beziehungsweise das Abdichtungssystem übertragen werden.

Günstig kann es ferner sein, wenn die Schutzelemente eine längs ihrer Erstreckung konstante Dicke aufweisen und/oder in sich flexibel ausgebildet sind. So können sie sich insbesondere auch großflächig an eine Innenseite des Dichtelements des Abdichtungssystems anlegen.

Um den Einsatz der Schutzvorrichtung in einem sterilen Raum zu ermöglichen, ist es günstig, wenn die Schutzvorrichtung aus einem sterilisierbaren Material hergestellt ist.

Günstigerweise ist die Schutzvorrichtung aus einem Kunststoff hergestellt. Aus einem Kunststoff lässt sich die Schutzvorrichtung insbesondere durch Spritzgießen auf einfache Weise herstellen. Hierzu ist vorteilhaft, wenn der Kunststoff vorzugsweise ein thermoplastischer Kunststoff ist.

Die eingangs gestellte Aufgabe wird bei einem chirurgischen Abdichtungssystem der eingangs beschriebenen Art erfindungsgemäß dadurch gelöst, dass die Schutzvorrichtung in Form einer der oben beschriebenen Schutzvorrichtungen ausgebildet ist.

Das Abdichtungssystem umfasst somit eine Schutzvorrichtung, die den Schutz des Dichtelements des Abdichtungssystems in optimaler Weise gewährleistet. Insbesondere weist somit das Abdichtungssystem auch die bereits oben im Zusammenhang mit bevorzugten Ausführungsformen von Schutzvorrichtungen beschriebenen Vorteile auf.

Günstig ist es, wenn das Dichtelement zum Abdichten von Schäften langgestreckter chirurgischer Instrumente beim Einführen in einen menschlichen oder tierischen Körper ausgebildet ist, eine Längsachse definiert und eine im Durchmesser veränderliche und quer oder im Wesentlichen quer zur Längsachse orientierte Öffnung aufweist, durch die ein Schaft einführbar ist. So kann insbesondere das Entweichen von Gas aus dem Körper eines Patienten verhindert werden, wenn ein Instrument durch die Trokarhülse in den Patientenkörper eingeführt wird beziehungsweise ist.

Vorteilhaft ist es, wenn das Dichtelement eine ringförmig geschlossene, flexible Wand umfasst, wenn die Wand einen ersten und einen zweiten, jeweils in sich geschlossenen Rand aufweist und wenn der erste Rand die Öffnung begrenzt. Mit einem solchen Dichtelement lassen sich insbesondere langgestreckte Schäfte, vorzugsweise mit einem kreisförmigen Querschnitt, optimal abdichten.

Um bei Bedarf die Schutzvorrichtung vom Dichtelement entfernen zu können, ist es günstig, wenn die Schutzvorrichtung mit dem Dichtelement lösbar verbindbar ist.

Gemäß einer weiteren bevorzugten Ausführungsform der Erfindung kann vorgesehen sein, dass am Grundkörper eine Verbindungseinrichtung zum Verbinden der Schutzvorrichtung mit dem chirurgischen Dichtelement oder dem chirurgischen Abdichtungssystem angeordnet ist und dass das Dichtelement zur Verbindungsvorrichtung korrespondierende Verbindungsglieder aufweist. Beispielsweise dann, wenn die Verbindungsglieder in Form von Verbindungsvorsprüngen ausgebildet sind, ist es günstig, wenn das Dichtelement hierzu korrespondierende Verbindungsaufnahmen aufweist.

Die nachfolgende Beschreibung bevorzugter Ausführungsformen der Erfindung dient im Zusammenhang mit den Zeichnungen der näheren Erläuterung. Es zeigen:
- Figur 1:: eine perspektivische Gesamtansicht eines chirurgischen Abdichtungssystems umfassend eine Trokarhülse;
- Figur 2A:: eine Seitenansicht einer chirurgischen Schutzvorrichtung in einer Grundstellung nach der Herstellung;
- Figur 2B:: eine Ansicht der die Grundstellung einnehmende Schutzvorrichtung in Richtung des Pfeils A in Figur 2A;
- Figur 2C:: eine perspektivische Ansicht der die Grundstellung einnehmende Schutzvorrichtung aus Figur 2A in der Grundstellung;
- Figur 3A:: eine Seitenansicht einer chirurgischen Schutzvorrichtung in einer Schutzstellung;
- Figur 3B:: eine Ansicht der die Schutzstellung einnehmende Schutzvorrichtung in Richtung des Pfeils B in Figur 3A;
- Figur 3C:: eine perspektivische Ansicht der die Schutzstellung einnehmende Schutzvorrichtung aus Figur 2A;
- Figur 4:: eine Längsschnittansicht einer in die Trokarhülse aus Figur 1 eingesetzten Dichtungseinheit mit Dichtelementhalterung, Dichtelement, Schutzvorrichtung und Schließventil;
- Figur 5:: eine Schnittansicht längs Linie 5-5 in Figur 4; und
- Figur 6:: eine Schnittansicht analog Figur 4 beim Einführen eines Obturators des Abdichtungssystems.

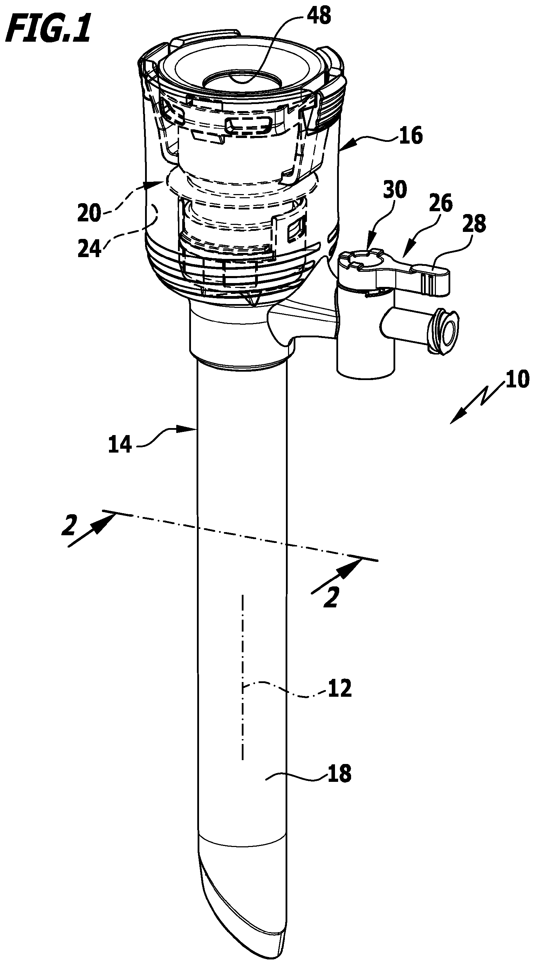

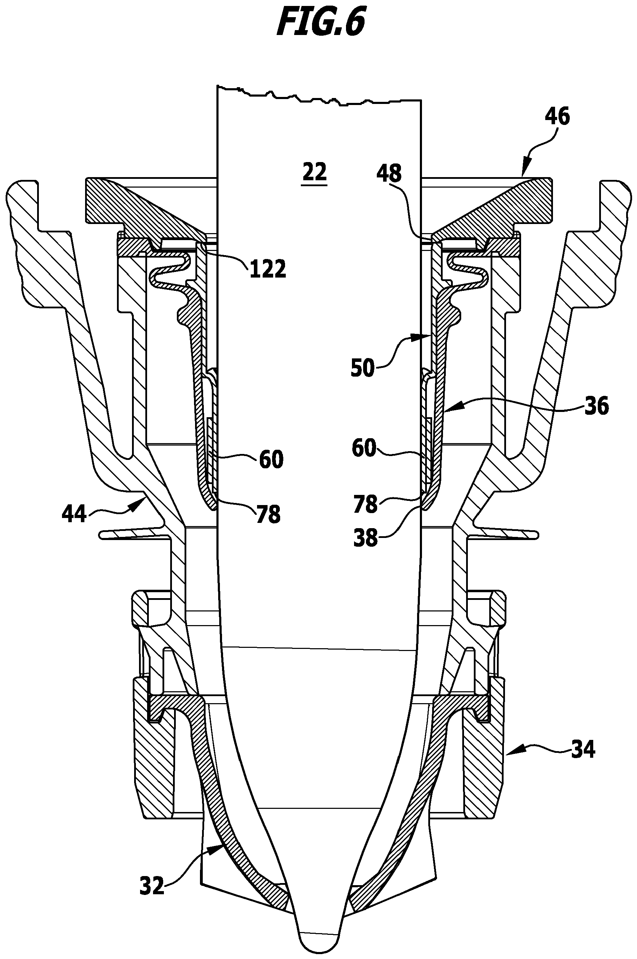

In den Figuren 1 bis 6 ist ein insgesamt mit dem Bezugszeichen 10 versehenes, ein chirurgisches Abdichtungssystem bildendes Trokarsystem schematisch dargestellt. Es umfasst eine eine Längsachse 12 definierende Trokarhülse 14 mit einem Dichtungsgehäuse 16 und einem sich von diesem weg in distaler Richtung erstreckenden Schaft 18, eine im Dichtungsgehäuse 16 angeordnete Dichtungsanordnung 20 sowie optional einen schematisch in Figur 6 dargestellten Obturator 22 zum Trennen und Aufweiten von Körpergewebe, welcher vor dem Einführen der Trokarhülse 14 in einen Patientenkörper in diese wie schematisch in Figur 6 dargestellt eingeschoben wird, um das Einführen der Trokarhülse 14 in den Patientenkörper zu erleichtern.

Die Trokarhülse 14 definiert im Inneren des Dichtungsgehäuses 16 eine Aufnahme 24 für die Dichtungsanordnung 20. Im Übergangsbereich zwischen dem Dichtungsgehäuse 16 und dem Schaft 18 ist ein quer zur Längsachse 12 abstehende Luer-Lock-Anschluss 26 angeordnet, über welchen eine Fluidverbindung in das Innere des Schafts 18 herstellbar ist. Am Luer-Lock-Anschluss 26 ist ferner ein mit einem Schwenkhebel 28 zur Betätigung ausgestattetes Schließventil 30 angeordnet.

Die Dichtungsanordnung 20 umfasst zwei Dichtungen, nämlich ein Kreuzschlitzventil 32, welches an einem mit einer Dichtelementhalterung 44 gekoppelten Haltering 34 gehalten ist, sowie ein Dichtelement 36, welches an seinem distalen Ende eine im Wesentlichen kreisförmige Einführöffnung 38 aufweist. Ein proximales, in Form eines Flansches 40 ausgebildetes Ende des Dichtelements 36 ist klemmend zwischen einer in proximaler Richtung weisenden Randfläche 42 der Dichtelementhalterung 44 und einem Deckel 46 gehalten. Der Deckel 46 weist eine kreisförmige Öffnung 48 auf, welche einen maximalen Außendurchmesser für in die Trokarhülse 14 einführbare Instrumentenschäfte definiert.

Die Dichtelementhalterung 44 ist, nach dem Verbinden mit dem Kreuzschlitzventil 32, Einsetzen des Dichtelements 36 von proximal her kommend und Festlegen desselben mittels des Deckels 46, von proximal her kommend in das Dichtungsgehäuse 46 einführbar und mit diesem rastend lösbar verbindbar.

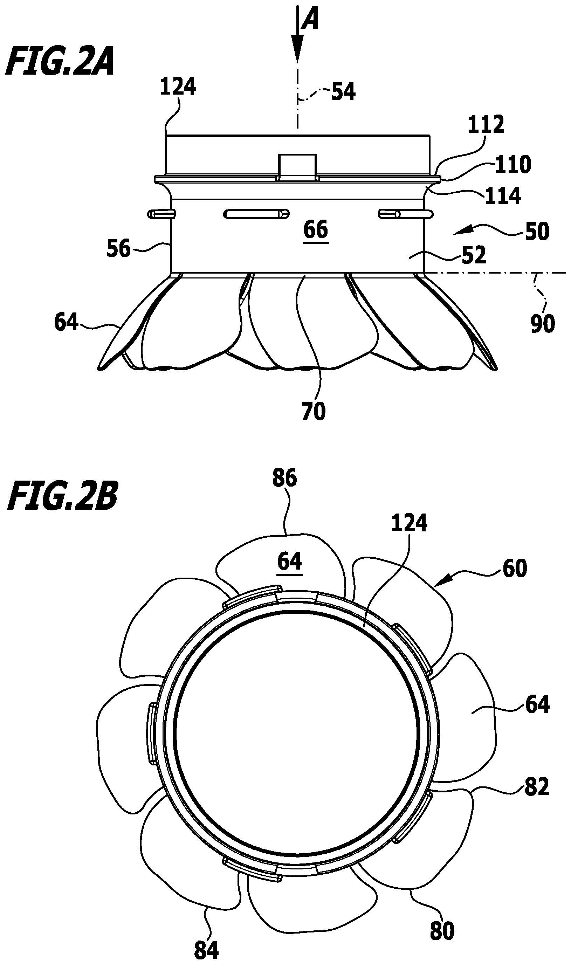

Zum Schutz des Dichtelements 36 dient eine insgesamt mit dem Bezugszeichen 50 bezeichnete Schutzvorrichtung. Diese ist in den Figuren 2A bis 2C in einer Grundstellung, welche sie direkt nach der Herstellung einnimmt, schematisch dargestellt. Insbesondere kann die Schutzvorrichtung 50 einstückig aus einem sterilisierbaren Kunststoff durch Spritzgießen hergestellt werden.

Die Schutzvorrichtung 50 umfasst einen Grundkörper 52, welcher im Wesentlichen in Form einer eine Längsachse 54 definierenden, kurzen Hülse 56 ausgebildet ist. An einer in distaler Richtung weisenden ringförmigen Randfläche 58 sind mehrere Schutzelemente 60, bei dem in den Figuren dargestellten Ausführungsbeispiel sind es acht Schutzelemente 60, gelenkig mit der Hülse 56 verbunden. Die Schutzelemente 60 sind in Form von dünnen Lamellen 62 ausgebildet, welche eine Dicke aufweisen, die maximal einer Wandstärke der Hülse 56 entspricht. Eine Außenseite 64 der Schutzelemente 60 ist von der Längsachse 54 weg weisend schwach konvex gekrümmt. Vorzugsweise entspricht eine Krümmung der Außenseite 64 einer Krümmung einer Außenseite 66 der Hülse 56.

In der Grundstellung direkt nach der Herstellung stehen die Schutzelemente 60 in radialer Richtung über die in distaler Richtung weisende Randfläche 58 von der Längsachse 54 weg weisend ab. So kann die Schutzvorrichtung 50 sauber und ohne die Schutzelemente 60 miteinander verbindende Grate durch Spritzgießen hergestellt werden. Alle Schutzelemente 60 sind identisch ausgebildet und gleichmäßig über den Umfang der Randfläche 58 verteilt angeordnet. Allerdings sind die Schutzelemente 60 voneinander etwas beabstandet.

Ein Abstand 68 erster Enden 70 der Schutzelemente 60, die mit der Randfläche 58 des Grundkörper 52 verbunden sind, entspricht mindestens der Dicke einer Wandstärke derselben, also einer Dicke der Hülse 56.

Die Schutzelemente 60 sind alle asymmetrisch bezogen auf eine Ebene 72 ausgebildet, welche die Längsachse 54 enthält. Jedes Schutzelement 60 weist zwei sich vom ersten Ende 70 weg erstreckende Ränder 74 und 76 auf, wobei der Rand 74 vom Schutzelement 60 weg weisend konvex gekrümmt ist und wobei der Rand 76 vom Schutzelement 60 weg weisend konkav gekrümmt ist. Alle konvex gekrümmten Ränder 74 der Schutzelemente 60 weisen in der Grundstellung auf die konkav gekrümmten Ränder 76 direkt benachbarter Schutzelemente 60 hin. In der Grundstellung überlappen sich die Schutzelemente 60 nicht, wie dies in den Figuren 2A bis 2C schematisch dargestellt ist. Die Ränder 74 und 76 benachbarter Schutzelemente 60 berühren sich zudem in der Grundstellung nicht, sondern weisen vielmehr einen Abstand voneinander auf, der mindestens der Größe des Abstands 68 entspricht.

Alle Schutzelemente 60 weisen ein zweites Ende 78 auf, welches vom Grundkörper 52 weg weist. Das zweite Ende 78 erstreckt sich zwischen den Rändern 74 und 76, die über Verrundungen 80 beziehungsweise 82 in das zweite Ende 78 übergehen. Das zweite Ende 78 definiert eine Endrandfläche 84, welche vom Grundkörper 52 weg weist. Sie kann geradlinig verlaufen oder optional vom Grundkörper 52 weg weisend schwach konvex gekrümmt sein, also eine schwach konvex gekrümmte Grundform aufweisen.

Ferner kann die Endrandfläche 84 optional einen vom Grundkörper 52 weg weisend konkav gekrümmten Randabschnitt 86 umfassen. Dieser kann sich insbesondere auf einer Länge der Endrandfläche 84 erstrecken, welche etwa der halben Länge zwischen den Verrundungen 80 und 82 entspricht.

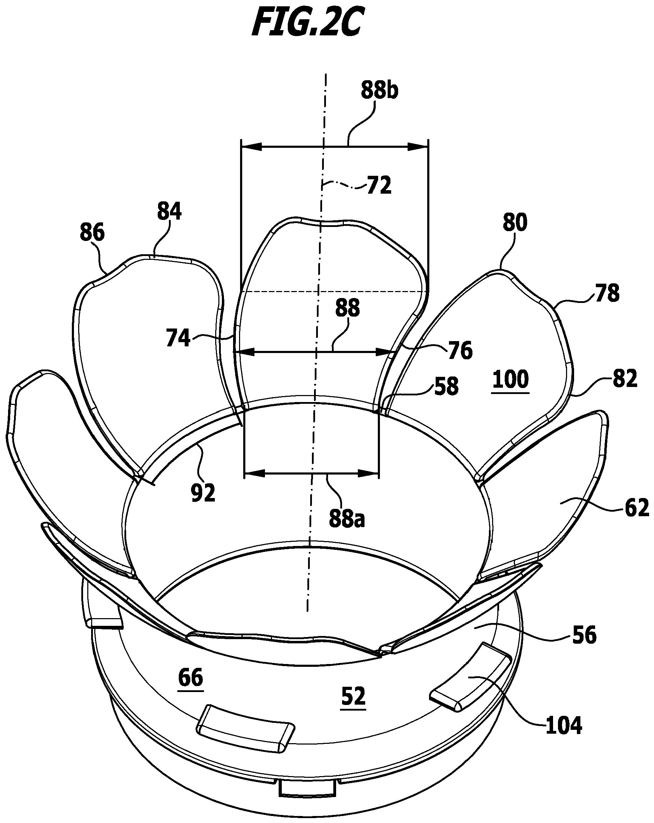

Eine Breite 88 der Schutzelemente 60 parallel zu einer die ersten Enden 70 enthaltenden Ebene 90 nimmt ausgehend vom ersten Ende 70 jedes Schutzelement 60 in Richtung auf das zweite Ende 78 hin zu. In Figur 2C ist schematisch die Breite 88a am ersten Ende 70 bezeichnet sowie eine maximale Breite 88b, die auch als ein Maximum der Breite 88 des Schutzelements 60 bezeichnet werden kann, wobei jedes Schutzelement zwischen dem ersten Ende 70 und der Endrandfläche 84 ein solches Maximum der Breite 88 aufweist. Die maximale Breite 88b jedes Schutzelements 60 ist dem zweiten Ende 78 näher als dem ersten Ende 70.

Die ersten Enden 70 der Schutzelemente 60 definieren einen Kreisbogenausschnitt 92, welcher einen Winkelbereich definiert, welcher einem Winkel entspricht, der etwas kleiner als ein Achtel von 360° bei dem in den Figuren 2A bis 2C dargestellten Ausführungsbeispiel ist.

Die Schutzelemente 60 sind in einem Übergangsbereich 94 zum Grundkörper 52 gelenkig und/oder scharnierartig mit diesem verbunden. Vorzugsweise ist jedes Schutzelement 60 mit dem Grundkörper 52 über eine Art Filmscharnier 96 verbunden. Die gelenkige Verbindung der Schutzelemente 60 mit dem Grundkörper 52 ermöglicht insbesondere eine Bewegung der Lamellen 62 von der Grundstellung in eine Schutzstellung durch Verschwenken der Lamellen in Richtung auf die Längsachse 54 hin.

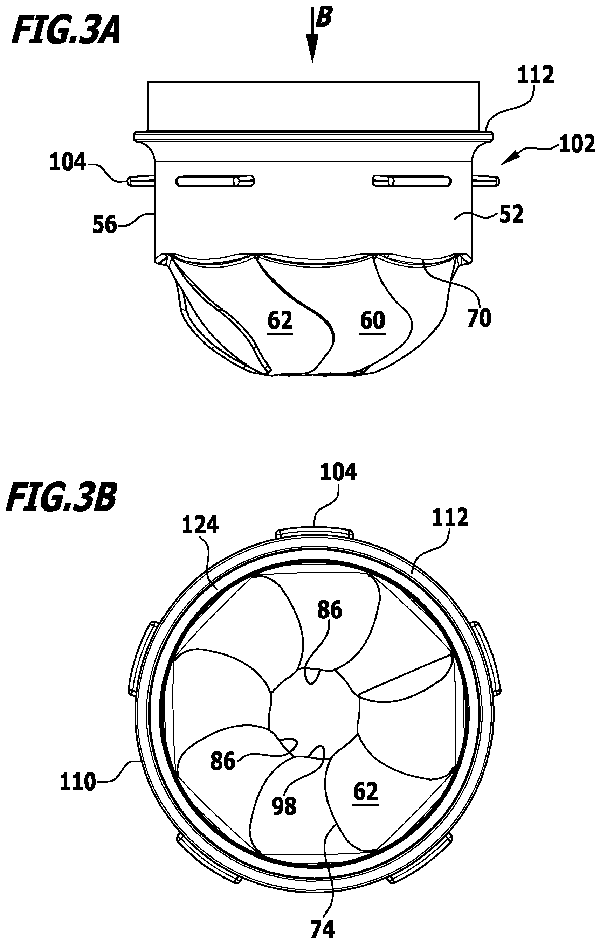

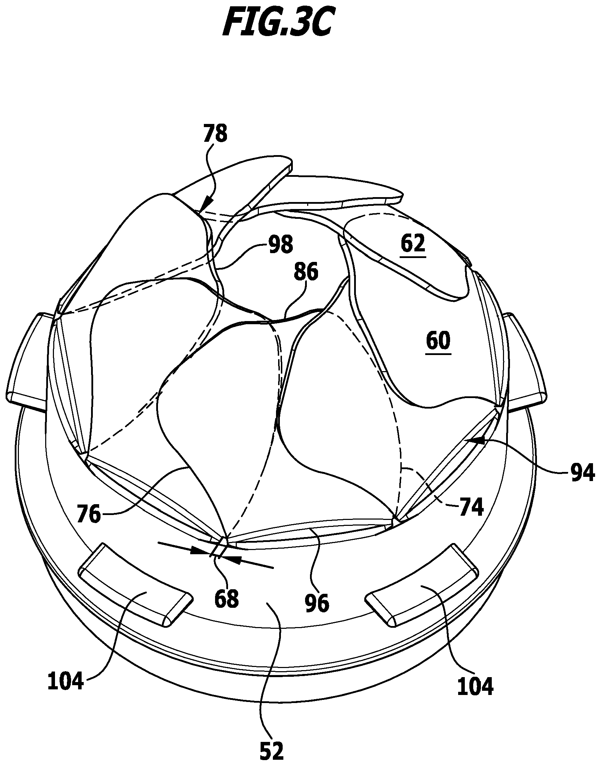

In den Figuren 3A bis 3C ist die Schutzvorrichtung 50 in einer von vielen möglichen Schutzstellungen schematisch dargestellt, in welcher sich die Schutzelemente 60 teilweise überlappen. Die schematisch dargestellte Schutzstellung definiert eine minimale Öffnung 98 der Schutzvorrichtung 50, die konzentrisch zur Längsachse 54 ausgebildet ist, wobei die Randabschnitte 86 der in der Schutzstellung in Richtung auf die Längsachse 54 hin verschwenkten Lamellen 62 jeweils im Wesentlichen konzentrisch zur Längsachse 54 orientiert sind und gemeinsam die im Wesentlichen kreisförmige Öffnung 98 bilden. In dieser Schutzstellung überlappen sich die Lamellen 62 derart, dass lediglich immer nur zwei Schichten von Lamellen 62 übereinander liegen. Dies ist insbesondere gut in Figur 3C zu erkennen. Die Schutzelemente 60 überlappen einander derart, dass die konkaven Ränder 76 auf den Außenseiten 64 der jeweils benachbarten Schutzelemente 60 anliegen, die konvexen Ränder 74 auf Innenseiten 100 der jeweils anderen, benachbarten Schutzelements 60.

Von proximal her kommend können insbesondere Instrumente mit Schäften durch die Schutzvorrichtung 50 eingeführt werden, die einen maximalen Außendurchmesser aufweisen, welcher einem Innendurchmesser der Hülse 56 entspricht. Beim Einführen der Schäfte werden die Lamellen 62 dann entsprechend nach außen verschwenkt, überlappen einander jedoch immer noch etwas in der beschriebenen Weise, so dass zumindest die Verrundungen 82 auf den Außenseiten 64 benachbarter Schutzelemente 60 anliegen.

Um eine definierte Position der Schutzvorrichtung 50 relativ zum Dichtelement 36 gewährleisten zu können, umfasst die Schutzvorrichtung 50 ferner eine Verbindungseinrichtung 102, welche insgesamt fünf in radialer Richtung von der Längsachse 54 weg weisende, flanschartige Verbindungsvorsprünge umfasst, die in Umfangsrichtung voneinander beabstandet sind. Sie sind korrespondierend zu Verbindungsaufnahmen 107 im Bereich einer wulstartigen Verdickung 106 auf einer Innenseite 108 des Dichtelements 36 ausgebildet.

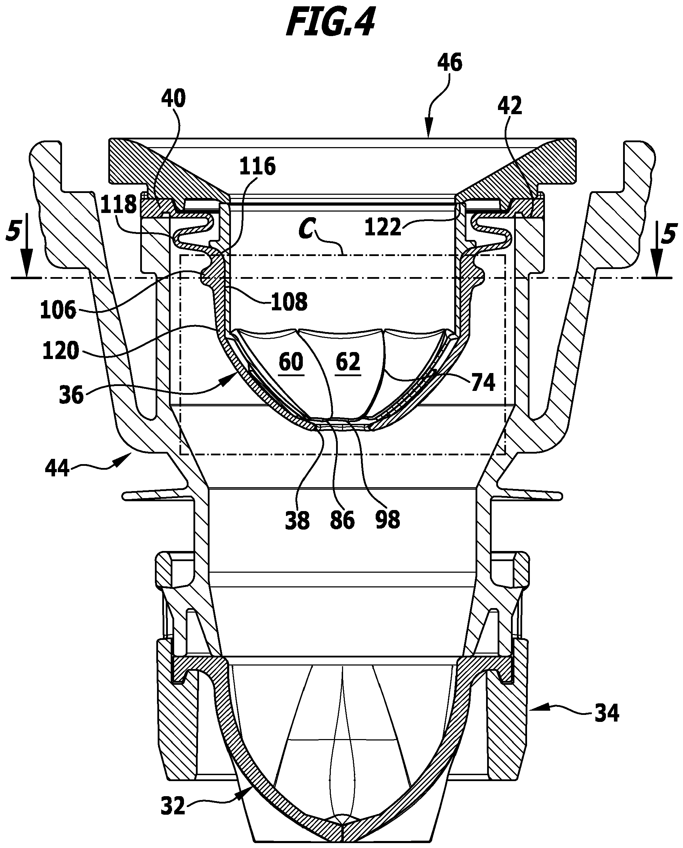

Proximalseitig der Verbindungsvorsprünge 104 ist ein in sich geschlossener ringförmiger Flansch 110 am Grundkörper 52 ausgebildet, welcher eine in proximaler Richtung weisende, ebene Ringfläche 112 umfasst sowie eine in distaler Richtung und etwas von der Längsachse 54 weg weisende, konkav gekrümmte Begrenzungsfläche 114. Die Begrenzungsfläche 114 liegt im montierten Zustand, wie schematisch in Figur 4 dargestellt, an einer konvex gekrümmten Ringfläche 116 des Dichtelements 36 an. Die Ringfläche 116 ist etwas proximalseitig der Verdickung 106 ausgebildet und bildet den Übergang in eine balgartige, von der Längsachse 54 weg weisende Ausstülpung 118 einer Wand 120 des Dichtelements 36. Ferner ist der Deckel mit einer in distaler Richtung weisenden Ringfläche 122 ausgestattet, welche an einer in proximaler Richtung weisenden Endfläche 124 des Grundkörpers 52 anliegt, wenn die Längsachse 54 des Grundkörpers 52 koaxial zur Längsachse 12 des Abdichtungssystems 10 ausgerichtet ist.

Die im Abdichtungssystem 10 angeordnete Schutzvorrichtung 50 ist in einer Schutzstellung, in welcher sich die Lamellen 62 wie in den Figuren 3A bis 3C schematisch dargestellt maximal überlappen, im unausgelenkten Zustand des Dichtelements 36 derart angeordnet, dass die Öffnung 98 konzentrisch zur Einführöffnung 38 positioniert ist und praktisch bis an diese heranreicht. So ist das Dichtelement 36 in seiner Grundstellung, in welcher die Einführöffnung 38 ungedehnt ist, optimal durch die Schutzvorrichtung 50 geschützt.

Wird ein chirurgisches Instrument oder, wie in Figur 6 schematisch dargestellt, ein Obturator 22 von proximal her kommend durch die Öffnung 48 des Deckels 46 ein- und durch das Dichtelement 36 sowie das Kreuzschlitzventil 32 hindurch geführt, wird mittels der Schutzvorrichtung 50 im Wesentlichen verhindert, dass das Instrument oder der Obturator mit dem Dichtelement 36 direkt in Kontakt treten kann. Eine Beschädigung des Dichtelements 36 kann so praktisch verhindert werden. Beim Einführen gleitet das Instrument an den Lamellen 62 auf und verschwenkt diese von der Längsachse 12 weg nach außen. Dabei nehmen die Lamellen 62, die mit ihren Außenseiten 64 an der Innenseite 108 des Dichtelements 36 anliegen, das Dichtelement 36 mit und dehnen es auf, wobei gleichzeitig die Einführöffnung 38 mit aufgedehnt wird.

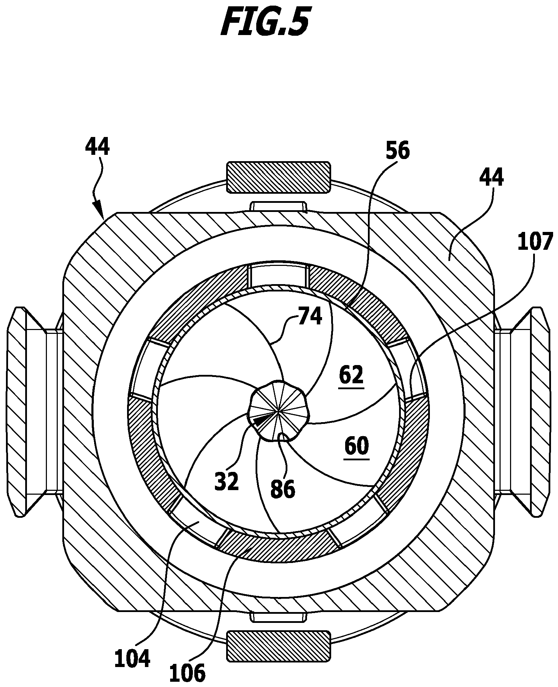

Beim Herausziehen des Instruments aus dem Abdichtungssystem 10 wirkt das vorgespannte Dichtelement 36 auf die Schutzelemente 60 ein und verschwenkt diese wieder zurück in die in den Figuren 4 und 5 schematisch dargestellte Schutzstellung, bei welcher die Einführöffnung 38 ungedehnt oder im Wesentlichen ungedehnt ist.

## Patentansprüche

1. Chirurgische Schutzvorrichtung (50) für ein chirurgisches, eine erweiterbare Einführöffnung (38) aufweisendes Dichtelement (36) eines chirurgischen, eine Trokarhülse (14) umfassenden Abdichtungssystems (10), welche Schutzvorrichtung (50) einen an der Trokarhülse (14), an einem Teil derselben oder am Dichtelement (36) anordenbaren, ringförmigen oder im Wesentlichen ringförmigen und eine Durchbrechung definierenden Grundkörper (52) mit mehreren in Umfangsrichtung angeordneten und parallel oder auf eine Längsachse (54) der Schutzvorrichtung (50) hin weisenden Schutzelementen (60) umfasst, wobei die Schutzvorrichtung (50) einstückig ausgebildet ist, wobei jedes der Schutzelemente (60) bezogen auf eine Ebene (72), welche eine vom Grundkörper (52) definierte Längsachse (54) enthält, asymmetrisch ausgebildet ist, wobei die Schutzelemente (60) erste Enden (70) aufweisen,
die am Grundkörper (52) angeordnet sind, und wobei jedes Schutzelement (60) zwei sich vom ersten Ende (70) weg erstreckende Ränder (74, 76) aufweist, wobei der eine Rand (74) vom Schutzelement (60) weg weisend konvex gekrümmt ist und wobei der andere Rand (76) vom Schutzelement (60) weg weisend konkav gekrümmt ist.

2. Chirurgische Schutzvorrichtung nach Anspruch 1, **dadurch gekennzeichnet, dass** der Grundkörper (52) im Wesentlichen in Form einer ringförmigen Hülse (56) ausgebildet ist und/oder dass die Schutzelemente (60) von der Längsachse (54) weg weisend schwach konvex gekrümmt sind.

3. Chirurgische Schutzvorrichtung nach Anspruch 1 oder 2, **dadurch gekennzeichnet, dass** die ersten Enden (70) am Grundkörper (52) an einer in distaler Richtung weisenden Randfläche (58) des Grundkörpers (52) angeordnet sind.

4. Chirurgische Schutzvorrichtung nach Anspruch 3, **dadurch gekennzeichnet, dass** die ersten Enden (70) der Schutzelemente (60) voneinander beabstandet sind und/oder dass ein Abstand (68) der ersten Enden (70) benachbarter Schutzelemente (60) mindestens einer Dicke der Schutzelemente (60) und/oder des Grundkörpers (52) entspricht und/oder dass die ersten Enden (70) der Schutzelemente (60) einen Kreisbogenausschnitt (92) definieren
und/oder dass die ersten Enden (70) der Schutzelemente (60) konzentrisch zur Längsachse (54) angeordnet sind und/oder dass die Schutzelemente (60) in einer Grundstellung nach der Herstellung in radialer Richtung über die in distaler Richtung weisende Randfläche (58) von der Längsachse (54) weg weisend abstehen.

5. Chirurgische Schutzvorrichtung nach einem der voranstehenden Ansprüche, **dadurch gekennzeichnet, dass** die Schutzelemente (60) in Form von Lamellen (62) geformt sind, welche eine Dicke aufweisen, welche maximal einer Dicke der den Grundkörper (52) definierenden Hülse (56) entspricht und/oder dass alle Schutzelemente (60) identisch ausgebildet sind.

6. Chirurgische Schutzvorrichtung nach einem der voranstehenden Ansprüche, **dadurch gekennzeichnet, dass** die konvex gekrümmten Ränder (74) der Schutzelemente (60) in der Grundstellung auf die konkav gekrümmten Ränder (76) direkt benachbarter Schutzelemente (60) hin weisen.

7. Chirurgische Schutzvorrichtung nach einem der Ansprüche 4 bis 6, **dadurch gekennzeichnet, dass** sich die Schutzelemente (60) in der Grundstellung nicht überlappen.

8. Chirurgische Schutzvorrichtung nach einem der voranstehenden Ansprüche, **dadurch gekennzeichnet, dass** die Schutzelemente (60) ein zweites, vom Grundkörper (52) weg weisendes Ende (78) aufweisen.

9. Chirurgische Schutzvorrichtung nach Anspruch 8, **dadurch gekennzeichnet, dass** das zweite Ende (78) sich zwischen den sich von den vom ersten Ende weg erstreckenden Rändern (74, 76) erstreckt und/oder dass das zweite Ende (78) eine Endrandfläche (84) definiert, welche im Wesentlichen eine vom Grundkörper (52) weg weisend schwach konvex gekrümmte Grundform aufweist, und dass die Endrandfläche (84) einen vom Grundkörper (52) weg weisend konkav gekrümmten Randabschnitt (86) umfasst.

10. Chirurgische Schutzvorrichtung nach einem der voranstehenden Ansprüche, **dadurch gekennzeichnet, dass** die Schutzelemente (60) in einem Übergangsbereich (94) zum Grundkörper (52) gelenkig und/oder scharnierartig mit dem Grundkörper (52) verbunden sind und/oder dass die Schutzelemente (60) und der Grundkörper (52) über ein Filmscharnier (96) miteinander verbunden sind.

11. Chirurgische Schutzvorrichtung nach einem der voranstehenden Ansprüche, **dadurch gekennzeichnet, dass** sich benachbarte Schutzelemente (60) in einer Schutzstellung teilweise überlappen.

12. Chirurgische Schutzvorrichtung nach Anspruch 11, **dadurch gekennzeichnet, dass** die Schutzelemente (60) in der Schutzstellung höchstens einen gemeinsamen maximalen Innendurchmesser definieren, der einem Innendurchmesser des Grundkörpers (52) entspricht und/oder dass die Schutzelemente (60) in der Schutzstellung einen minimalen Innendurchmesser definieren, wenn sie sich maximal überlappen, und dass dabei die konkav gekrümmten Randabschnitte (86) der Endrandflächen (84) konzentrisch oder im Wesentlichen konzentrisch zur Längsachse (54) ausgerichtet und dieser maximal angenähert sind und/oder dass sich die Schutzelemente (60) in der Schutzstellung, unabhängig davon, welchen Innendurchmesser sie dabei mit ihren zweiten Enden (78) definieren, nur derart überlappen, dass ausschließlich einander benachbarte Schutzelemente (60) übereinander liegen und/oder dass der konkav gekrümmte Rand (76) der Schutzelemente (60) den konvex gekrümmten Rand (74) auf einer von der Längsachse (54) weg weisenden Außenseite (64) des direkt benachbarten Schutzelements (60) überdeckt oder umgekehrt.

13. Chirurgische Schutzvorrichtung nach einem der voranstehenden Ansprüche, **gekennzeichnet durch** eine am Grundkörper (52) angeordnete Verbindungseinrichtung (102) zum Verbinden der Schutzvorrichtung (50) mit dem chirurgischen Dichtelement (36) oder dem chirurgischen Abdichtungssystem (10).

14. Chirurgische Schutzvorrichtung nach einem der voranstehenden Ansprüche, **dadurch gekennzeichnet, dass** die Schutzelemente (60) eine längs ihrer Erstreckung konstante Dicke aufweisen und/oder in sich flexibel ausgebildet sind und/oder dass die Schutzvorrichtung (50) aus einem sterilisierbaren Material hergestellt ist und/oder dass die Schutzvorrichtung (50) aus einem Kunststoff hergestellt ist.

15. Chirurgisches Abdichtungssystem (10) umfassend eine Trokarhülse (14), ein an der Trokarhülse (14) oder einem Teil derselben gehaltenes chirurgisches, eine erweiterbare Einführöffnung (38) aufweisendes Dichtelement (36) zum Abdichten der Einführöffnung (38) beim Einführen eines chirurgischen Instruments (22) und eine chirurgische Schutzvorrichtung (50) für das Dichtelement (36), **dadurch gekennzeichnet, dass** die Schutzvorrichtung (50) in Form einer Schutzvorrichtung (50) nach einem der voranstehenden Ansprüche ausgebildet ist.

## Claims

1. Surgical protective device (50) for a surgical sealing element (36) of a surgical sealing system (10) comprising a trocar sleeve (14), the surgical sealing element (36) having an expandable insertion opening (38), said protective device (50) comprising a ring-shaped or substantially ring-shaped main body (52) adapted to be arranged on the trocar sleeve (14), on a part thereof or on the sealing element (36) and defining a through-opening with a plurality of protective elements (60) arranged in the circumferential direction and extending parallel to or pointing towards a longitudinal axis (54) of the protective device (50), wherein the protective device (50) is of one-piece construction, wherein each of the protective elements (60) is configured asymmetrically in relation to a plane (72) containing a longitudinal axis (54) defined by the main body (52), wherein the protective elements (60) have first ends (70) which are arranged on the main body (52), and wherein each protective element (60) has two edges (74, 76) extending away from the first end (70), wherein the one edge (74) is convexly curved facing away from the protective element (60), and wherein the other edge (76) is concavely curved facing away from the protective element (60).

2. Surgical protective device in accordance with claim 1, **characterized in that** the main body (52) is configured substantially in the form of a ring-shaped sleeve (56) and/or **in that** the protective elements (60) are slightly convexly curved facing away from the longitudinal axis (54).

3. Surgical protective device in accordance with claim 1 or 2, **characterized in that** the first ends (70) are arranged on the main body (52) on an edge surface (58) of the main body (52) that faces in the distal direction.

4. Surgical protective device in accordance with claim 3, **characterized in that** the first ends (70) of the protective elements (60) are spaced from one another and/or **in that** a spacing (68) between the first ends (70) of adjacent protective elements (60) corresponds at least to a thickness of the protective elements (60) and/or of the main body (52) and/or **in that** the first ends (70) of the protective elements (60) define a circular arc segment (92)
and/or **in that** the first ends (70) of the protective elements (60) are arranged concentrically with the longitudinal axis (54) and/or **in that** in an initial position after manufacture, the protective elements (60) project in the radial direction facing away from the longitudinal axis (54) over the edge surface (58) facing in the distal direction.

5. Surgical protective device in accordance with any one of the preceding claims, **characterized in that** the protective elements (60) are formed in the shape of lamellas (62) having a thickness corresponding at the most to a thickness of the sleeve (56) defining the main body (52) and/or **in that** all of the protective elements (60) are identically constructed.

6. Surgical protective device in accordance with any one of the preceding claims, **characterized in that** the convexly curved edges (74) of the protective elements (60), in the initial position, face towards the concavely curved edges (76) of directly adjacent protective elements (60).

7. Surgical protective device in accordance with any one of claims 4 to 6, **characterized in that** the protective elements (60) do not overlap in the initial position.

8. Surgical protective device in accordance with any one of the preceding claims, **characterized in that** the protective elements (60) have a second end (78) facing away from the main body (52).

9. Surgical protective device in accordance with claim 8, **characterized in that** the second end (78) extends between the edges (74, 76) extending away from the first end and/or **in that** the second end (78) defines an end edge surface (84) essentially having a slightly convexly curved basic shape facing away from the main body (52), and **in that** the end edge surface (84) has a concavely curved edge section (86) facing away from the main body (52).

10. Surgical protective device in accordance with any one of the preceding claims, **characterized in that** the protective elements (60) are connected in an area of transition (94) to the main body (52) in an articulated and/or hinge-like manner to the main body (52) and/or **in that** the protective elements (60) and the main body (52) are connected to one another by a film hinge (96).

11. Surgical protective device in accordance with any one of the preceding claims, **characterized in that** adjacent protective elements (60) overlap partially in a protective position.

12. Surgical protective device in accordance with claim 11, **characterized in that** the protective elements (60), in the protective position, define at the most a joint maximum inner diameter corresponding to an inner diameter of the main body (52) and/or **in that** the protective elements (60), in the protective position, define a minimal inner diameter when they overlap to the maximum extent, and **in that** the concavely curved edge sections (86) of the end edge surfaces (84) are then aligned concentrically or substantially concentrically with the longitudinal axis (54) and are maximally close thereto and/or **in that** the protective elements (60), in the protective position, irrespective of which inner diameter they define with their second ends (78), only overlap to such an extent that solely adjacent protective elements (60) lie on top of each other and/or **in that** the concavely curved edge (76) of the protective elements (60) covers the convexly curved edge (74) on an outer side (64) of the directly adjacent protective element (60) that faces away from the longitudinal axis (54) or vice versa.

13. Surgical protective device in accordance with any one of the preceding claims, **characterized by** a connecting device (102) arranged on the main body (52) for connecting the protective device (50) to the surgical sealing element (36) or to the surgical sealing system (10).

14. Surgical protective device in accordance with any one of the preceding claims, **characterized in that** the protective elements (60) have a constant thickness along their extent and/or are of inherently flexible construction and/or **in that** the protective device (50) is produced from a sterilizable material and/or **in that** the protective device (50) is produced from a plastic material.

15. Surgical sealing system (10) comprising a trocar sleeve (14), a surgical sealing element (36) held on the trocar sleeve (14) or on a part thereof and having an expandable insertion opening (38), for sealing the insertion opening (38) when inserting a surgical instrument (22), and a surgical protective device (50) for the sealing element (36), **characterized in that** the protective device (50) is configured in the form of a protective device (50) in accordance with any one of the preceding claims.

## Revendications

1. Dispositif de protection chirurgical (50) pour un élément d'étanchéité chirurgical (36) présentant une ouverture d'introduction expansible (38), d'un système d'étanchéité chirurgical (10) comprenant une chemise de trocart (14), ledit dispositif de protection (50) comportant un corps de base (52), qui peut être agencé sur la chemise de trocart (14), sur une partie de celle-ci ou sur l'élément d'étanchéité (36), qui est de forme annulaire ou sensiblement de forme annulaire et définit un passage, et qui comporte plusieurs éléments de protection (60) agencés dans la direction périphérique et orientés parallèlement ou de manière à être dirigés vers un axe longitudinal (54) du dispositif de protection (50), et le dispositif de protection (50) étant réalisé d'un seul tenant et de façon telle
que chacun des éléments de protection (60) soit d'une configuration asymétrique par rapport à un plan (72), qui renferme un axe longitudinal (54) défini par le corps de base (52),
que les éléments de protection (60) présentent des premières extrémités (70), qui sont agencées sur le corps de base (52), et
que chaque élément de protection (60) présente deux bords (74, 76) issus de la première extrémité (70),
que ledit un bord (74) soit d'une courbure convexe dans la direction s'éloignant de l'élément de protection (60), et
que l'autre bord (76) soit d'une courbure concave dans la direction s'éloignant de l'élément de protection (60) .

2. Dispositif de protection chirurgical selon la revendication 1, **caractérisé en ce que** le corps de base (52) est réalisé sensiblement sous la forme d'un fourreau (56) de forme annulaire, et/ou **en ce que** les éléments de protection (60) sont d'une courbure sensiblement convexe dans la direction s'éloignant de l'axe longitudinal (54).

3. Dispositif de protection chirurgical selon la revendication 1 ou la revendication 2, **caractérisé en ce que** les premières extrémités (70) sont agencées sur le corps de base (52) au niveau d'une surface de bordure (58) du corps de base (52), qui est dirigée en direction distale.

4. Dispositif de protection chirurgical selon la revendication 3, **caractérisé en ce que** les premières extrémités (70) des éléments de protection (60) sont espacées les unes des autres, et/ou **en ce qu'**une distance d'espacement (68) entre les premières extrémités (70) d'éléments de protection (60) voisins correspond au moins à l'épaisseur des éléments de protection (60) et/ou du corps de base (52), et/ou **en ce que** les premières extrémités (70) des éléments de protection (60) définissent un secteur d'arc de cercle (92),
et/ou **en ce que** les premières extrémités (70) des éléments de protection (60) sont agencées concentriquement à l'axe longitudinal (54), et/ou **en ce que** les éléments de protection (60), dans une position de base après la fabrication, dépassent, en direction radiale, de la surface de bordure (58) dirigée en direction distale, en s'éloignant de l'axe longitudinal (54) .

5. Dispositif de protection chirurgical selon l'une des revendications précédentes, **caractérisé en ce que** les éléments de protection (60) sont réalisés sous forme de lamelles (62), qui présentent une épaisseur, qui, au maximum, correspond à une épaisseur du fourreau (56) définissant le corps de base (52), et/ou **en ce que** tous les éléments de protection (60) sont d'une configuration identique.

6. Dispositif de protection chirurgical selon l'une des revendications précédentes, **caractérisé en ce que** les bords (74) de courbure convexe des éléments de protection (60) sont orientés, dans la position de base, en direction des bords (76) de courbure concave d'éléments de protection (60) directement voisins.

7. Dispositif de protection chirurgical selon l'une des revendications 4 à 6, **caractérisé en ce que** les éléments de protection (60) ne se chevauchent pas dans la position de base.

8. Dispositif de protection chirurgical selon l'une des revendications précédentes, **caractérisé en ce que** les éléments de protection (60) présentent une deuxième extrémité (78) éloignée du corps de base (52).

9. Dispositif de protection chirurgical selon la revendication 8, **caractérisé en ce que** la deuxième extrémité (78) s'étend entre les bords (74, 76) issus de la première extrémité, et/ou **en ce que** la deuxième extrémité (78) définit une surface de bordure d'extrémité (84), qui présente une forme de base de courbure légèrement convexe en s'éloignant du corps de base (52), et **en ce que** la surface de bordure d'extrémité (84) comporte un tronçon de bordure (86) de courbure concave en s'éloignant du corps de base (52).

10. Dispositif de protection chirurgical selon l'une des revendications précédentes, **caractérisé en ce que** les éléments de protection (60) sont reliés au corps de base (52), dans une zone de transition (94) au corps de base (52), de manière articulée et/ou à la manière d'une charnière, et/ou **en ce que** les éléments de protection (60) et le corps de base (52) sont reliés mutuellement par l'intermédiaire d'une charnière à film (96).

11. Dispositif de protection chirurgical selon l'une des revendications précédentes, **caractérisé en ce que** des éléments de protection (60) voisins se chevauchent partiellement dans une position de protection.

12. Dispositif de protection chirurgical selon la revendication 11, **caractérisé en ce que** dans la position de protection, les éléments de protection (60) définissent tout au plus un diamètre intérieur maximal commun, qui correspond à un diamètre intérieur du corps de base (52), et/ou **en ce que** les éléments de protection (60) définissent, dans la position de protection, un diamètre intérieur minimal, lorsqu'ils se chevauchent au maximum, et **en ce qu'**à cette occasion les tronçons d'extrémité (86) courbés de manière concave, des surfaces de bordure d'extrémité (84), sont orientés de manière concentrique ou sensiblement concentrique à l'axe longitudinal (54) et sont rapprochés au maximum de celui-ci, et/ou **en ce que** les éléments de protection (60), dans la position de protection, indépendamment du diamètre intérieur qu'ils définissent à cette occasion avec leurs deuxièmes extrémités (78), se chevauchent mutuellement uniquement de façon à ce que des éléments de protection (60) voisins soient superposés, et/ou que le bord (76) de courbure concave des éléments de protection (60) recouvre le bord (74) de courbure convexe, sur un côté extérieur (64) orienté dans une direction s'éloignant de l'axe longitudinal (54), de l'élément de protection (60) directement voisin, ou inversement.

13. Dispositif de protection chirurgical selon l'une des revendications précédentes, **caractérisé par** un dispositif de liaison (102) agencé sur le corps de base (52), pour relier le dispositif de protection (50) à l'élément d'étanchéité chirurgical (36) ou au système d'étanchéité chirurgical (10).

14. Dispositif de protection chirurgical selon l'une des revendications précédentes, **caractérisé en ce que** les éléments de protection (60) présentent une épaisseur constante le long de leur étendue et/ou sont réalisés flexibles en soi, et/ou **en ce que** le dispositif de protection (50) est fabriqué en un matériau pouvant être stérilisé, et/ou **en ce que** le dispositif de protection (50) est fabriqué en une matière plastique.

15. Système d'étanchéité chirurgical (10) comprenant une chemise de trocart (14), un élément d'étanchéité chirurgical (36) avec une ouverture d'introduction expansible (38), qui est maintenu sur la chemise de trocart (14) ou une partie de celle-ci et est destiné à assurer l'étanchéité de l'ouverture d'introduction (38) lors de l'introduction d'un instrument chirurgical (22), et un dispositif de protection chirurgical (50) pour l'élément d'étanchéité (36), **caractérisé en ce que** le dispositif de protection (50) est réalisé sous la forme d'un dispositif de protection (50) selon l'une des revendications précédentes.
